# EUROPEAN PATENT APPLICATION

(11) **EP 2 727 544 A1**
(43) Date of publication of application: **07.05.2014**
(21) Application number: 13191589.4
(22) Date of filing: 05.11.2013
(51) Int. Cl.: A61B 17/22, A61M 37/00, A61N 7/02, A61B 19/00

(54) **Catheter systems and methods**

(30) Priority: 05.11.2012 US 201261722731 P
(71) Applicant: Ekos Corporation, Bothell, WA 98011 (US)
(72) Inventor: Genstler, Curtis, Snohomish, WA 98280 (US); Hansmann, Douglas R., Bainbridge Island, WA 98110 (US); Wolniewicz III, Raymond M., Redmond, WA 98052 (US)
(74) Representative: Tischner, Oliver

(57) **Abstract**

An ultrasound catheter system can be configured to provide therapeutic ultrasound and imaging. An ultrasound catheter system can comprise a catheter having a distal portion. In several embodiments, a first ultrasonic element can be disposed in the distal portion of the catheter and can be configured to emit a first ultrasonic energy to enhance delivery of a therapeutic compound. In some embodiments, a second ultrasonic element can be disposed in the distal portion of the catheter and can be configured to convert a second ultrasonic energy that is reflected from an imaging site into an electrical signal.

## Description

### CROSS-REFERENCE TO RELATED APPLICATIONS

This application claims the priority benefit of U.S. Provisional Application No. 61/722,731, filed November 5, 2012.

### BACKGROUND

### Field

The present invention relates generally to medical devices and, more particular, in some embodiments a medical device configured to image a portion of a treatment site and to deliver ultrasonic energy and a therapeutic compound to a treatment site.

### Description of Related Art

Several medical applications use ultrasonic energy. For example, U.S. Patent Numbers 4,821,740, 4,953,565, and 5,007,438 disclose the use of ultrasonic energy to enhance the effect of various therapeutic compounds. An ultrasonic catheter can be used to deliver ultrasonic energy and a therapeutic compound to a treatment site in a patient's body. Such an ultrasonic catheter typically includes an ultrasound assembly configured to generate ultrasonic energy and a fluid delivery lumen for delivering the therapeutic compound to the treatment site.

As taught in U.S. Patent Number 6,001,069, such ultrasonic catheters can be used to treat human blood vessels that have become partially or completely occluded by plaque, thrombi, emboli, or other substances that reduce the blood carrying capacity of the vessel. To remove or reduce the occlusion, the ultrasonic catheter is used to deliver solutions containing dissolution compounds directly to the occlusion site. Ultrasonic energy generated by the ultrasound assembly enhances the therapeutic effect of the dissolution compounds. For example, in one application of such an ultrasonic catheter, an ultrasound-enhanced thrombolytic therapy dissolves blood clots in arteries and veins in the treatment of diseases such as peripheral arterial occlusion or deep vein thrombosis. In such applications, ultrasonic energy enhances thrombolysis with agents such as urokinase, tissue plasminogen activator ("TPA"), and the like.

U.S. Patent Application Publication Number 2008/0319376 discloses a method and apparatus that can be used to treat intracranial hemorrhages and/or a subarachnoid hemorrhage with ultrasonic energy in conjunction with a therapeutic compound and/or light treatment.

Ultrasonic catheters can also be used to enhance gene therapy at a treatment site within the patient's body. For example, U.S. Patent Number 6,135,976 discloses an ultrasonic catheter having one or more expandable sections capable of occluding a section of a body lumen, such as a blood vessel. A gene therapy composition is then delivered to the occluded vessel through the catheter fluid delivery lumen. Ultrasonic energy generated by the ultrasound assembly is applied to the occluded vessel, thereby enhancing the delivery of a genetic composition into the cells of the occluded vessel.

Ultrasonic catheters can also be used to enhance delivery and activation of light activated drugs or drugs used to treat cancers and/or tumors. For example, U.S. Patent Number 6,176,842 discloses methods for using an ultrasonic catheter to treat biological tissues by delivering a light activated drug to the biological tissues and exposing the light activated drug to ultrasound energy.

There are also intravascular ultrasound devices ("IVUS"), which can be used to graphically depict a vessel and or a target site in a patient. For example, IVUS can be used to measure thickness of the atherosclerotic plaque along the length of the diseased area in a vessel and at specific radial positions around its circumference.

### SUMMARY

In certain medical procedures, it is desirable to combine therapeutic techniques and devices with techniques and devices that can provide information about the treatment site (e.g., the target site) and/or progress of a treatment protocol. In one arrangement, the therapeutic assembly can include a device that is configured to provide a therapeutic compound and/or ultrasonic energy to the treatment site. For example, in certain embodiments, therapeutic compounds and ultrasound are used to dissolve blockages and/or hematomas. The assembly can also be able to provide information about the treatment site by using ultrasound imaging and/or another imaging technology. In some embodiments, the assembly can use optical sensing, Doppler, electromagnetic, and/or other techniques for gathering information about the treatment site. The therapeutic assembly can be an ultrasound catheter system.

In several embodiments, an ultrasound catheter system can comprise a catheter having at least one ultrasonic element and a control system configured to drive the ultrasonic element to generate ultrasonic energy. The control system can also be configured to receive and process information from an imaging device.

Some embodiments include methods of operating an ultrasonic catheter system. In some embodiments, methods include advancing a catheter with at least one ultrasonic element and/or at least one imaging device to a treatment site in a patient (e.g., in a vascular system or tissue). Methods can include imaging the treatment site and/or a location inside the patient that is within one inch of the treatment site. Some methods include driving the at least one ultrasonic element to generate ultrasonic energy. Several methods include delivering a therapeutic compound to the treatment site through the catheter.

Several embodiments include performing treatment at a treatment site and sensing a condition at the treatment site. Performing the treatment can include delivering ultrasound to the treatment site. Sensing a condition at the treatment site can include delivering ultrasound, sensing a response of tissue to the ultrasound, and correlating the response to a condition. Some embodiments include adjusting treatment at the treatment site in response to the sensed condition. Several systems are configured to perform one or more methods described herein.

In some embodiments, an ultrasound catheter system can be configured to provide therapeutic ultrasound and imaging ultrasound. An ultrasound catheter system can comprise a catheter having a treatment portion. In certain embodiments, the treatment portion is position at a distal portion of the catheter. A first ultrasonic element can be disposed in the treatment portion of the catheter and can be configured to emit a first ultrasonic energy to enhance delivery of a therapeutic compound. A second ultrasonic element can be disposed in the treatment portion of the catheter and can be configured to convert a second ultrasonic energy that is reflected from an imaging site into an electrical signal.

In some embodiments, the imaging device can comprise a transducer (e.g., an ultrasonic element). The ultrasound catheter system can comprise a characterization device capable of receiving information from the transducer. The characterization device can be electrically coupled to the transducer and/or be configured to wirelessly communicate with the transducer. The characterization device can be configured to analyze the imaging site.

In several embodiments, an ultrasound catheter system can be configured to provide therapeutic ultrasound. The ultrasound catheter system can comprise a catheter having a treatment portion. A therapeutic assembly can comprise a first ultrasonic element disposed in the treatment portion of the catheter and configured to emit a first ultrasonic energy to enhance delivery of a therapeutic compound. An imaging assembly can comprise a signal emitter and a signal sensor. The signals can be ultrasonic energy, any imaging technology disclosed herein, or any other suitable imaging technology. The signal emitter and the signal sensor can be disposed in the treatment portion of the catheter. The imaging assembly can be configured to analyze a treatment site by emitting a first signal and/or sensing a second signal. The second signal can be related to the first signal. In some embodiments, the first ultrasonic element, the signal emitter, and/or the signal sensor can be covered by a cavitation promoting surface.

Some methods are related to operating an ultrasonic catheter. Several methods include obtaining a catheter comprising a distal portion having a first ultrasonic element and a second ultrasonic element. Some methods include enhancing delivery of a therapeutic compound by emitting a first ultrasonic energy radially outward from the first ultrasonic element towards a treatment site. Several methods include imaging the treatment site by emitting a second ultrasonic energy radially outward from the second ultrasonic element towards the treatment site; reflecting at least a portion of the second ultrasonic energy from the treatment site; and/or converting the portion of the second ultrasonic energy into an electrical signal. The imaging can occur from the catheter before the enhancing occurs from the catheter. The enhancing and the imaging can occur concurrently (e.g., simultaneously). The first ultrasonic energy can be at least three times greater than the second ultrasonic energy.

### BRIEF DESCRIPTION OF THE DRAWINGS

Various embodiments are depicted in the accompanying drawings for illustrative purposes, and should in no way be interpreted as limiting the scope of the embodiments. In addition, various features of different disclosed embodiments can be combined to form additional embodiments, which are part of this disclosure.

Figure 1 is a schematic illustration of certain features of an example ultrasonic catheter system, according to some embodiments.

Figure 2 illustrates a cross-sectional view of the ultrasonic a catheter of Figure 1 taken along line 2-2, according to some embodiments.

Figure 3 is a schematic illustration of an elongate inner core configured to be positioned within the central lumen of the catheter illustrated in Figure 2, according to some embodiments.

Figure 4 illustrates a cross-sectional view of the elongate inner core of Figure 3 taken along line 4-4, according to some embodiments.

Figure 5 is a schematic wiring diagram illustrating a technique for electrically connecting five groups of ultrasound radiating members to form an ultrasound assembly, according to some embodiments.

Figure 6 is a schematic wiring diagram illustrating a technique for electrically connecting one of the groups of Figure 5, according to some embodiments.

Figure 7A is a schematic illustration of the ultrasound assembly of FIG. 5 housed within the inner core of Figure 4.

Figure 7B is a cross-sectional view of the ultrasound assembly of Figure 7A taken along line 7B-7B.

Figure 7C is a cross-sectional view of the ultrasound assembly of Figure. 7A taken along line 7C-7C.

Figure 7D is a side view of an ultrasound assembly center wire twisted into a helical configuration.

Figure 8 illustrates is a schematic illustration an embodiment of the imaging device of Figure 1.

Figure 8A illustrates is a schematic illustration of an embodiment imaging device of Figure 1.

Figure 9 illustrates a longitudinal, cross-sectional view of selected components of an ultrasound catheter assembly, according to some embodiments.

Figure 10A is a schematic illustration of an ultrasonic catheter configured for insertion within the cranial cavity.

Figure 10B is an enlarged detail view of the distal end of the ultrasonic catheter shown in Figure 10A.

Figure 10C is an enlarged detail view of the proximal end of the ultrasonic catheter shown in Figure 10A.

Figure 10D is a schematic illustration of a stylet that can inserted into the ultrasonic catheter shown in Figure 10A.

Figure 10E is a schematic illustration of ultrasonic core with an imaging device that can inserted into the ultrasonic catheter shown in Figure 10A.

### DETAILED DESCRIPTION

Although certain embodiments and examples are disclosed herein, inventive subject matter extends beyond the examples in the specifically disclosed embodiments to other alternative embodiments and/or uses, and to modifications and equivalents thereof. Thus, the scope of the claims appended hereto is not limited by any of the particular embodiments described herein. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent and/or that all operations need to be performed. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein. No feature, benefit, advantage, structure, or step disclosed herein is essential or indispensable.

The drawings illustrate certain embodiments and are not intended to be limiting. The drawings can be semi-diagrammatic and not to scale. For clarity of presentation and discussion, some portions of and/or dimensions in the drawings are shown greatly exaggerated.

As described above, according to certain embodiments, it is desired to combine therapeutic devices and/or methods with a device and/or method having the ability to measure and/or image characteristics of the treatment site. Examples of a therapeutic device and/or method include ultrasonic catheters that can be used to enhance the therapeutic effects of therapeutic compounds at a treatment site within a patient's body. Examples of a device and/or method to measure and/or image characteristics of the treatment site include ultrasound imaging catheters and/or devices that use optical, IR, temperature, and/or Doppler measurement techniques. Examples of advantages of such systems include the ability to monitor the progression or efficacy of treatment and/or correct placement of the device at the treatment site. In other embodiments, the device has the ability to adjust the treatment based on the measured and/or imaged information. Preferred embodiments having certain of these features and advantages are described herein.

As used herein, the term "catheter" is used broadly, includes its ordinary meaning, and further includes an elongate flexible tube configured to be inserted into the body of a patient, such as into a body part, cavity, duct, or vessel. As used herein, the term "end" is used broadly, includes its ordinary meaning, and further encompasses a region generally, such that "proximal end" includes "proximal region," and "distal end" includes "distal region".

As used herein, the term "therapeutic compound" refers broadly, without limitation, and in addition to its ordinary meaning, to a drug, medicament, dissolution compound, genetic material, or any other substance capable of effecting physiological functions. Additionally, a mixture including substances such as these items is also encompassed within this definition of "therapeutic compound." Examples of therapeutic compounds include thrombolytic compounds, anti-thrombosis compounds, and other compounds used in the treatment of vascular occlusions and/or blood clots, including compounds intended to prevent or reduce clot formation. Exemplary therapeutic compounds include, but are not limited to, heparin, urokinase, streptokinase, tPA, rtPA, BB-10153 (manufactured by British Biotech, Oxford, UK), plasmin, IIbIIa inhibitors, desmoteplase, and factor VIIa. Therapeutic compound also refers to drugs that treat cancer (e.g., oncological drugs, sonodynamic drugs) and drugs for treating tumors and gliomas in the brain. The methods and apparatuses described above can be used to treat tumors and gliomas instead of (or in addition to) blood clots.

As used herein, the terms "ultrasonic energy," "ultrasound," and "ultrasonic" refer broadly, without limitation, and in addition to their ordinary meaning, to mechanical energy transferred through longitudinal pressure or compression waves. Ultrasonic energy can be emitted as continuous or pulsed waves, depending on the parameters of a particular application. Additionally, ultrasonic energy can be emitted in waveforms having various shapes, such as sinusoidal waves, triangle waves, square waves, or other wave forms. Ultrasonic energy includes sound waves. In certain embodiments, the ultrasonic energy referred to herein has a frequency between about 20 kHz and about 20 MHz. For example, in one embodiment, the ultrasonic energy has a frequency between about 500 kHz and about 20 MHz. In another embodiment, the ultrasonic energy has a frequency between about 1 MHz and about 3 MHz. In yet another embodiment, the ultrasonic energy has a frequency of about 2 MHz. In certain embodiments described herein, the average acoustic power of the ultrasonic energy is between about 0.01 watts and 300 watts. In one embodiment, the average acoustic power is about 15 watts.

As expounded herein, ultrasonic energy is sometimes used to enhance the delivery and/or effect of a therapeutic compound. For example, in the context of treating vascular occlusions, ultrasonic energy has been shown to increase enzyme mediated thrombolysis by enhancing the delivery of thrombolytic agents into a thrombus, where such agents lyse the thrombus by degrading the fibrin that forms the thrombus. The thrombolytic activity of the agent is enhanced in the presence of ultrasonic energy in the thrombus. However, it should be appreciated that the invention should not be limited to the mechanism by which the ultrasound enhances treatment unless otherwise stated. In other applications, ultrasonic energy has also been shown to enhance transfection of gene-based drugs into cells, and augment transfer of chemotherapeutic drugs into tumor cells. Ultrasonic energy delivered from within a patient's body has been found to be capable of producing non-thermal effects that increase biological tissue permeability to therapeutic compounds by up to or greater than an order of magnitude.

Certain features and aspects of the ultrasonic catheters disclosed herein may also find utility in applications where the ultrasonic energy itself provides a therapeutic effect. Examples of such therapeutic effects include preventing or reducing stenosis and/or restenosis; tissue ablation, abrasion or disruption; promoting temporary or permanent physiological changes in intracellular or intercellular structures; and rupturing micro-balloons or micro-bubbles for therapeutic compound delivery. Further information about such methods can be found in U.S. Patents Numbers 5,261,291 and 5,431,663, the entire disclosure of which are hereby incorporated herein by reference. Further information about using cavitation to produce biological effects can be found in U.S. Patent RE36,939.

U.S. Patent Number 8,192,391, which is hereby incorporated in its entirety herein by reference, discloses an ultrasound system that that can be used to treat vascular obstructions such as clots in the peripheral vascular system and/or in the pulmonary arteries (see e.g., U.S. Application Publication 2012/0289889, the entirety of which is hereby incorporated by reference herein.) Some systems described in U.S. Patent Number 8,192,391 are similar to the EkoSonic Endovascular system sold by EKOS Corporation. U.S. Patent Application Publication Number 2012/0059285, which is hereby incorporated in its entirety herein by reference, discloses an ultrasound system that can be used to treat an intracranial hemorrhage and/or a subarachnoid hemorrhage with ultrasonic energy in conjunction with a therapeutic compound. As used herein, the term "intracranial hemorrhage" encompasses both intracerebral hemorrhage and intraventricular hemorrhage.

Although several embodiments will be disclosed in the context of the embodiments of U.S. Patent Number 8,192,391 and U.S. Patent Application Publication Number 2012/0059285, it should be appreciated that the present application is not limited to these embodiments. Specifically, other embodiments may be directed to other treatment sites such as, for example, the treatment of other vessels of the vascular system (e.g., the coronary arteries) and/or portions of the body (e.g., tumors outside the vascular system).

Several embodiments include an imaging device that is used to measure and/or image a targeted treatment site. In some embodiments, the device includes an ultrasound imaging device. In some embodiments, the device can use optical sensing, Doppler, electromagnetic, and/or other techniques for gathering information about the treatment site, identifying a treatment site, and/or diagnosing medical conditions.

In several embodiments, the imaging device (e.g., an ultrasound imaging device) can be used in combination with a treatment device (e.g., devices described in U.S. Patent Number 8,192,391 and U.S. Patent Application Publication Number 2012/0059285). For example, in one arrangement, the treatment device of U.S. Patent Number 8,192,391 and/or U.S. Patent Application Publication Number 2012/0059285 can be used with an ultrasound imaging device to determine the location of a clot in an obstructed vessel. In such an arrangement, the imaging device can aid a operator in determining if the therapeutic device is properly positioned within the clot (or other treatment site). The imaging device can also allow the operator to identify the clot and/or determine if the device is being advanced into the clot. In this manner, the operator can determine if the tip of the catheter is being advanced into the vessel wall instead of into the clot. In this manner, the operator can avoid dissecting or otherwise damaging the blood vessel and/or another part of a patient. The imaging device can also be used to determine if the obstruction is being removed or decreasing in size during treatment. Some embodiments include delivering ultrasonic therapy (e.g., ultrasonic-enhanced drug delivery) to a treatment site while imaging the treatment site via ultrasonic imaging. The imaging device can also be used to determine if treatment should be stopped and/or modified.

In several embodiments, the imaging device can be used to position the ultrasound device in a hematoma. For example, the device of U.S. Patent Application Publication Number 2012/0059285 can be used to treat an intracranial hemorrhage and/or a subarachnoid hemorrhage. The imaging device can be used to position the device within the hematoma and to determine if the hematoma is being removed or decreasing in size during treatment. The imaging device can also be used to determine if treatment should be stopped and/or modified.

In some embodiments, the treatment device and the imaging device can be used at other treatment sites such as, for example, tumors, and/or organs of the body. The imaging device can also be used to position the device and/or to guide treatment.

As noted above, in some embodiments, the imaging device can comprise an ultrasound imaging catheter. In several embodiments, an imaging catheter can be inserted into and/or integrated into an ultrasound treatment catheter of U.S. Patent Number 8,192,391 and/or U.S. Patent Application Publication Number 2012/0059285. For example, the ultrasound core of U.S. Patent Number 8,192,391 can be removed and the imaging catheter can be placed within the drug delivery catheter. In several embodiments, the treatment catheter can include one or more ultrasound elements that are configured for imaging. In some embodiments, the ultrasound elements can be operated and one or more sensors or receivers can be placed outside the patient such that the ultrasound elements are configured for imaging inside of the patient. In several embodiments, the ultrasound elements can be placed inside a portion of the body (e.g., inside veins, inside arteries, inside the brain, inside an organ, inside tissue) to enable imaging of the treatment site or of another site. By using triangulation, the receivers or sensors can be used to provide imaging and/or location data.

As will be described below, the ultrasound catheter can include one or more ultrasound radiating members positioned therein. Such ultrasound radiating members can comprise a transducer (e.g., a PZT transducer), which can be configured to convert electrical energy into ultrasonic energy. In such embodiments, the PZT transducer is excited by specific electrical parameters (herein "power parameters" that cause it to vibrate in a way that generates ultrasonic energy). (PZT refers to lead zirconium titanate, which commonly has piezoelectric effects.)

Many embodiments disclosed herein are compatible with a wide variety of ultrasound catheters, several examples of which are disclosed in U.S. Patent Application Publication Number 2004/0024347 A1 (published 5 February 2004; discloses catheters especially well-suited for use in the peripheral vasculature) and U.S. Patent Application Publication Number 2005/0215942 A1 (published 29 September 2005; discloses catheters especially well-suited for use in the cerebral vasculature).

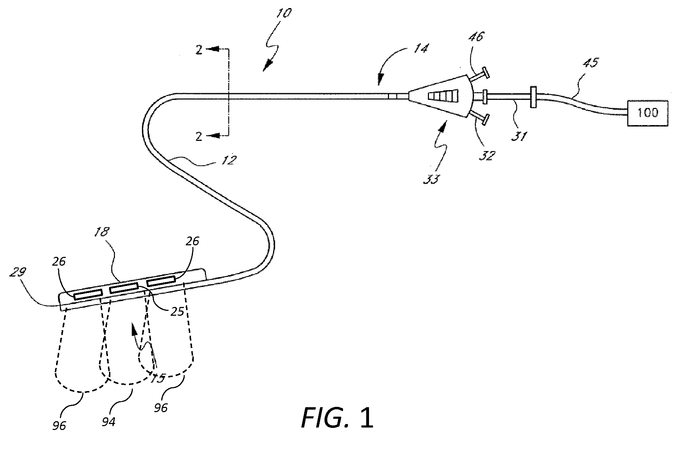

With reference to the illustrated embodiments, Figure 1 illustrates an ultrasonic catheter 10 configured for use in a patient's vasculature and/or in another portion of the patient's body. For example, in certain applications the ultrasonic catheter 10 is used to treat long segment peripheral arterial occlusions, such as those in the vascular system of the leg, while in other applications the ultrasonic catheter 10 is used to treat occlusions in the small vessels of the neurovasculature or other portions of the body (e.g., other distal portions of the vascular system). In other embodiments, the catheter can be used to treat pulmonary embolisms. Thus, the dimensions of the catheter 10 can be adjusted based on the particular application for which the catheter 10 is to be used.

The ultrasonic catheter 10 generally comprises a multi-component, elongate flexible tubular body 12 having a proximal region 14 and a distal region 15. The tubular body 12 can include a therapeutic and imaging section 18 located in the distal region 15 of the catheter 10. In some embodiments, the therapeutic and imaging section 18 includes at least one imaging device 25 and at least one treatment device 26. In the illustrated arrangement, a first treatment device 26 can be located distally relative to the imaging device 25 and a second treatment device 26 can be located proximally relative to the imaging device 25. The imaging device 25 can be an ultrasound imaging device or any other imaging system described herein. The treatment device 26 can be an ultrasound treatment device or any other treatment and/or therapeutic device described herein.

In some embodiments, the treatment device 26 is used to provide both therapy and imaging. For example, the ultrasound radiating member can radiate a first ultrasound to image a treatment site (e.g., to characterize the treatment site) and can radiate a second ultrasound to provide therapy to a treatment site (e.g., to enhance the delivery and/or effect of a therapeutic compound). In some embodiments, the first ultrasound and the second ultrasound have the same characteristics. In some embodiments, the first ultrasound and the second ultrasound have different characteristics.

The tubular body 12 and other components of the catheter 10 can be manufactured in accordance with a variety of techniques. Suitable materials and dimensions can be selected based on the natural and anatomical dimensions of the treatment site and on the desired percutaneous access site.

For example, in several embodiments, the proximal region 14 of the tubular body 12 comprises a material that has sufficient flexibility, kink resistance, rigidity, and structural support to push the therapeutic and imaging section 18 through the patient's vasculature to a treatment site. Examples of such materials include, but are not limited to, extruded polytetrafluoroethylene ("PTFE"), polyethylenes ("PE"), polyamides, and other similar materials. In certain embodiments, the proximal region 14 of the tubular body 12 is reinforced by braiding, mesh, or other constructions to provide increased kink resistance and pushability. For example, in certain embodiments, nickel titanium or stainless steel wires are placed along or incorporated into the tubular body 12 to reduce kinking.

In several embodiments, the therapeutic and imaging section 18 of the tubular body 12 optionally comprises a material that (a) is thinner than the material comprising the proximal region 14 of the tubular body 12, or (b) has a greater acoustic transparency than the material comprising the proximal region 14 of the tubular body 12. Thinner materials generally have greater acoustic transparency than thicker materials. Suitable materials for the therapeutic and imaging section 18 include, but are not limited to, high or low density polyethylenes, urethanes, nylons, and the like. In certain modified embodiments, the therapeutic and imaging section 18 is formed from the same material or a material of the same thickness as the proximal region 14.

One or more fluid delivery lumens can be incorporated into the tubular body 12. For example, in some embodiments, a central lumen passes through the tubular body 12.

A catheter can be configured to have one or more ultrasound radiating members positioned therein. For example, in certain embodiments, an ultrasound radiating member is positioned within the therapeutic and imaging section 18 of the tubular body, while in other embodiments, a plurality of ultrasound radiating members are coupled to an assembly that is passed into the central lumen. In either case, the one or more ultrasound radiating members can be electrically coupled to a control system 100 via a cable 45. In some embodiments, the outer surface of the therapeutic and imaging section 18 can include a cavitation promoting surface configured to enhance and/or promote cavitation at the treatment site.

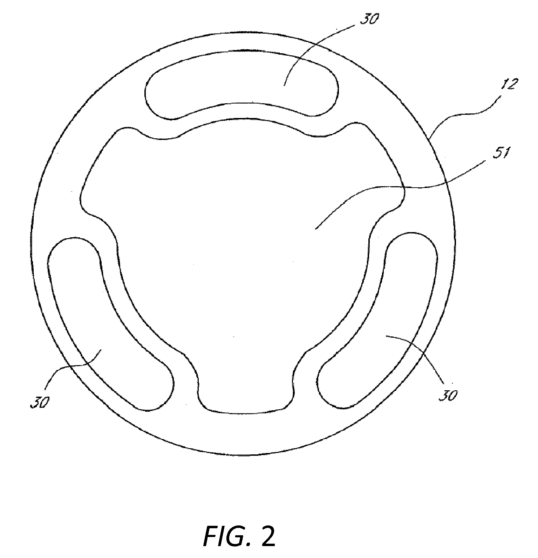

Figure 2 illustrates a cross section of the tubular body 12 taken along line 2-2 in Figure 1. In the embodiment illustrated in Figure 2, three fluid delivery lumens 30 are incorporated into the tubular body 12. In other embodiments, more or fewer fluid delivery lumens can be incorporated into the tubular body 12. The arrangement of the fluid delivery lumens 30 can provide a hollow central lumen 51 passing through the tubular body 12. The cross-section of the tubular body 12, as illustrated in Figure 2, can be substantially constant along the length of the catheter 10 (shown in Figure 1).

In certain embodiments, the central lumen 51 has a minimum diameter greater than about 0.030 inches. In another embodiment, the central lumen 51 has a minimum diameter greater than about 0.037 inches. In some embodiments, the fluid delivery lumens 30 have dimensions of about 0.026 inches wide by about 0.0075 inches high, although other dimensions may be used in other applications.

As described above, the central lumen 51 can extend through the length of the tubular body 12. As illustrated in Figure 1, the central lumen 51 can have a distal exit port 29 and a proximal access port 31. The proximal access port 31 can form part of the backend hub 33, which can be attached to the proximal region 14 of the catheter 10. The backend hub can further comprise a cooling fluid fitting 46, which can be hydraulically connected to the central lumen 51. The backend hub 33 can also comprise a therapeutic compound inlet port 32, which can be in hydraulic connection with the fluid delivery lumens 30, and which can be hydraulically coupled to a source of therapeutic compound via a hub such as a Luer fitting.

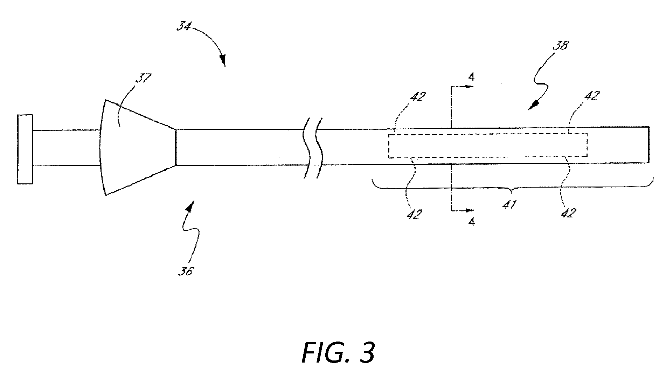

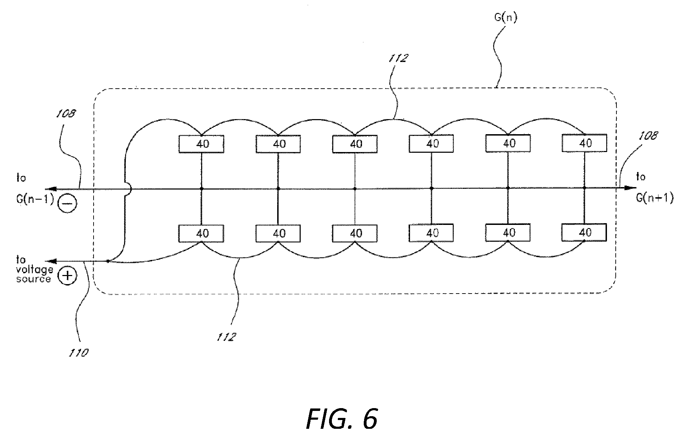

The central lumen 51 can be configured to receive an elongate inner core 34 (shown in Figure 3). The elongate inner core 34 can comprise a proximal region 36 and a distal region 38. A proximal hub 37 can be fitted on the inner core 34 at one end of the proximal region 36. One or more ultrasound radiating members can be positioned within an inner core energy delivery section 41 located within the distal region 38. The ultrasound radiating members 40 (shown in Figure 6) can form an ultrasound assembly 42.

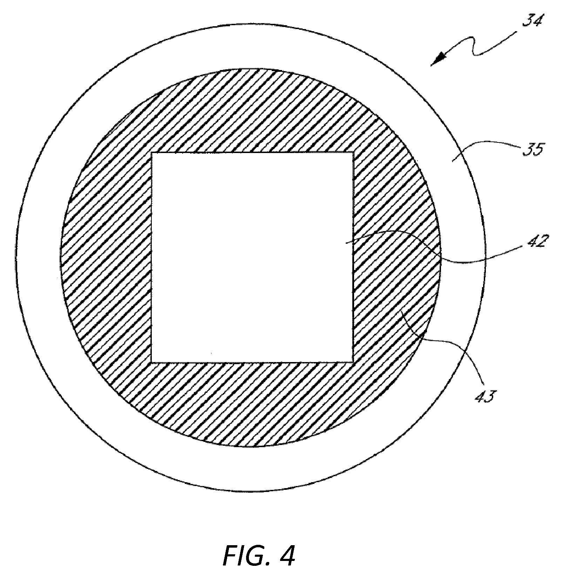

As shown in the cross-section illustrated in Figure 4, which is taken along lines 4-4 in Figure 3, the inner core 34 can have a cylindrical shape, with an outer diameter that permits the inner core 34 to be inserted into the central lumen 51 (shown in Figure 2) of the tubular body 12 (shown in Figure 2) via the proximal access port 31 (shown in Figure 1). The inner core 34 can comprise a cylindrical outer body 35 that houses the ultrasound assembly 42, which can form part of the treatment device 26. The ultrasound assembly 42 can comprise wiring and ultrasound radiating members, such that the ultrasound assembly 42 is capable of radiating ultrasonic energy from the energy delivery section 41 (shown in Figure 3) of the inner core 34. Referring now to Figures 1 and 4, the ultrasound assembly 42 can be electrically connected to the backend hub 33, where the inner core 34 can be connected to a control system 100 via the cable 45.

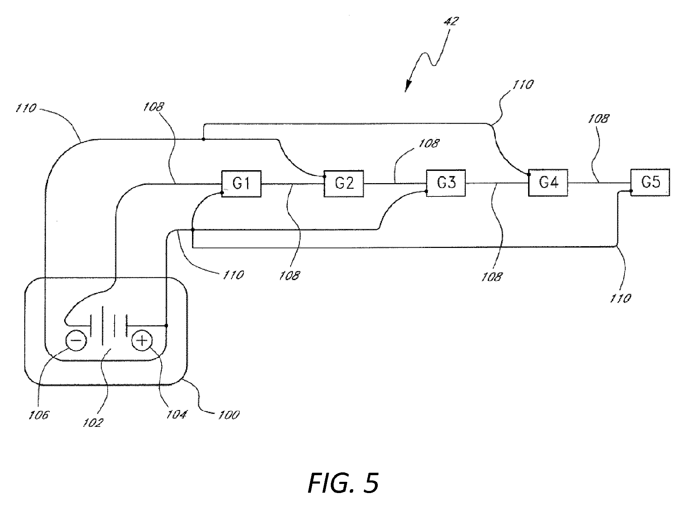

Figure 5 is a schematic wiring diagram illustrating one technique for connecting five groups of ultrasound radiating members to form the ultrasound assembly 42 of the treatment device 26. The ultrasound assembly 42 can comprise a plurality of ultrasound radiating members that are divided into one or more groups. For example, as illustrated in Figure 5, the ultrasound assembly 42 comprises five groups (labeled as G1, G2, G3, G4, and G5) of ultrasound radiating members that are electrically connected to each other. The five groups can also be electrically connected to the control system 100. These 5 groups can form portions of the first and second treatment devices 26 described above.

The control system 100 can comprise, among other things, a voltage source 102. The voltage source 102 can comprise a positive terminal 104 and a negative terminal 106. The negative terminal 106 can be connected to common wire 108, which can connect the five groups G1-G5 of ultrasound radiating members in series. The positive terminal 104 can be connected to a plurality of lead wires 110, which each connect to one of the five groups G1-G5 of ultrasound radiating members 40 (shown in Figure 6). Thus, under this configuration, each of the five groups, G1-G5, can be connected to the positive terminal 104 via one of the lead wires 110, and to the negative terminal 106 via the common wire 108. The control circuitry can be configured as part of the control system 100 and can include circuits, control routines, controllers, etc. configured to vary one or more power parameters used to drive ultrasound radiating members 40. As illustrated in Figure 6, each group can comprise a plurality of ultrasound radiating members 40, which can be electrically coupled by a wire 112.

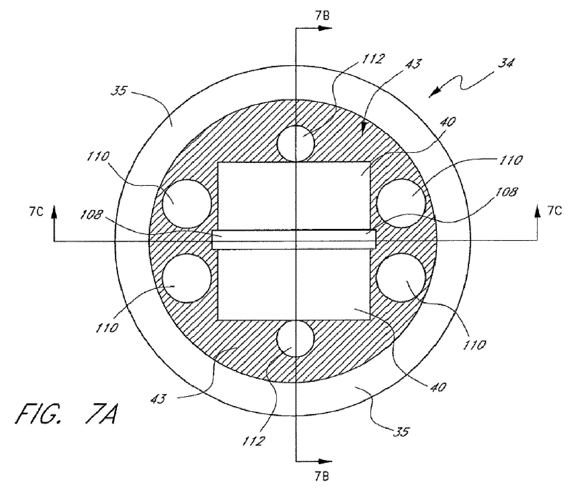

Figure 7A illustrates one preferred technique for arranging the components of the ultrasound assembly 42 (as schematically illustrated in Figure 5) into the inner core 34 (as schematically illustrated in Figure 4). Figure 7A is a cross-sectional view of the ultrasound assembly 42 taken within group G1 in Figure 5, as indicated by the presence of four lead wires 110. For example, if a cross-sectional view of the ultrasound assembly 42 was taken within group G4 in Figure 5, only one lead wire 310 would be present (that is, the one lead wire connecting group G5).

Referring still to Figure 7A, the common wire 108 comprises an elongate, flat piece of electrically conductive material in electrical contact with a pair of ultrasound radiating members 40. Each of the ultrasound radiating members 40 is also in electrical contact with a positive contact wire 312. Because the common wire 108 is connected to the negative terminal 106, and the positive contact wire 312 is connected to the positive terminal 104, a voltage difference can be created across each ultrasound radiating member 40. Lead wires 110 are preferably separated from the other components of the ultrasound assembly 42, thus preventing interference with the operation of the ultrasound radiating members 40 as described above. For example, in one preferred embodiment, the inner core 34 is filled with an insulating potting material 43, thus deterring unwanted electrical contact between the various components of the ultrasound assembly 42.

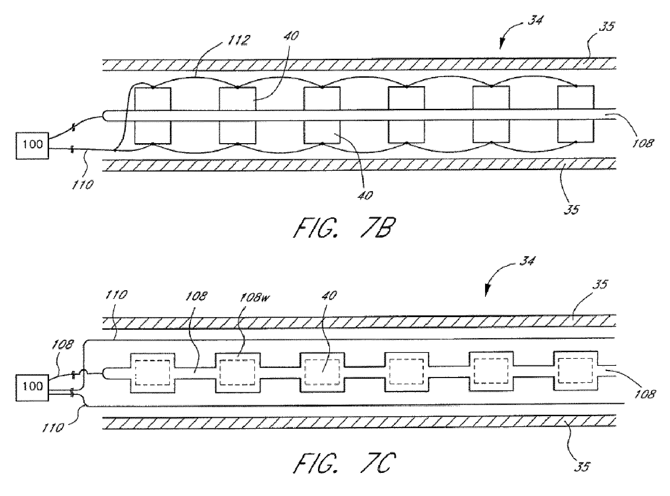

Figures 7B and 7C illustrate cross sectional views of the inner core 34 of Figure 7A taken along lines 7B-7B and 7C-7C, respectively. As illustrated in Figure 7B, the ultrasound radiating members 40 are mounted in pairs along the common wire 108. The ultrasound radiating members 40 are connected by positive contact wires 112, such that substantially the same voltage is applied to each ultrasound radiating member 40. As illustrated in Figure 7C, the common wire 108 preferably comprises wide regions 108W upon which the ultrasound radiating members 40 can be mounted, thus reducing the likelihood that the paired ultrasound radiating members 40 will short together. In certain embodiments, outside the wide regions 108W, the common wire 108 may have a more conventional, rounded wire shape.

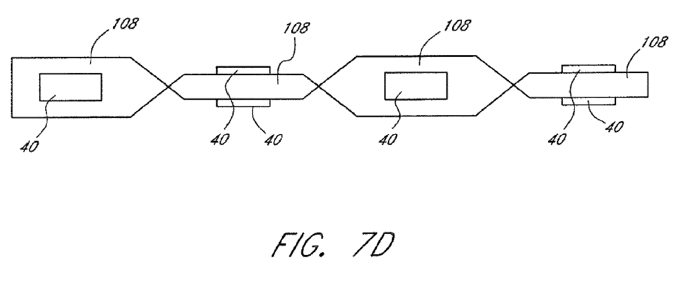

In a modified embodiment, such as illustrated in Figure 7D, the common wire 108 is twisted to form a helical shape before being fixed within the inner core 34. In such embodiments, the ultrasound radiating members 40 are oriented in a plurality of radial directions, thus enhancing the radial uniformity of the resulting ultrasonic energy field.

One of ordinary skill in the art will recognize that the wiring arrangement described above can be modified to allow each group G1, G2, G3, G4, G5 to be independently powered. Specifically, by providing a separate power source within the control system 100 for each group, each group can be individually turned on or off, or can be driven with an individualized power. This provides the advantage of allowing the delivery of ultrasonic energy to be "turned off" in regions of the treatment site where treatment is complete, thus preventing deleterious or unnecessary ultrasonic energy to be applied to the patient.

Further details and additional embodiments of a catheter having features according to the embodiment of Figures 1-7D can be found in U.S. Patent No. 8,192,391, the entirety of which, is hereby incorporated by reference herein.

As described above, in the illustrated embodiment, the catheter 10 can include an imaging device 25 integrated into the treatment portion of the catheter. In the illustrated arrangement, the imaging device 25 can be integrated into the ultrasound core 34. However, as noted above, in several embodiments, an imaging catheter can be inserted into and/or integrated into the ultrasound treatment catheter 10. For example, the ultrasound core 34 can be removed and an imaging catheter can be placed within the drug delivery catheter.

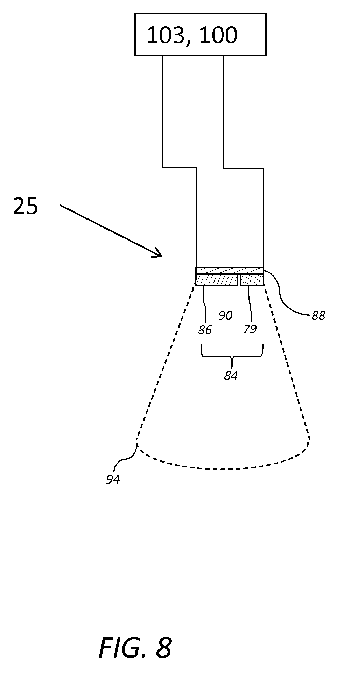

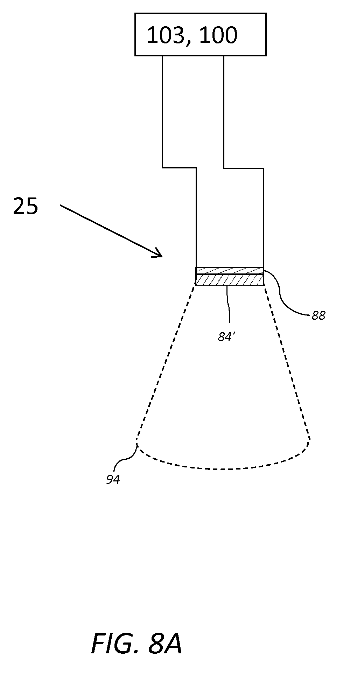

Figure 8 is a schematic illustration of the imaging device 25 of the catheter 10. In the illustrated embodiment, the imaging device can include a transducer 86 and a backing 88. In other embodiments, the backing 88 can be omitted. The backing 88 can be located radially inward relative to the transducer 86. The transducer 86 can be an ultrasonic transducer, an ultrasonic radiating member, and/or an ultrasonic element that can be configured to generate a second ultrasonic energy that is reflected from an imaging site into an electrical signal. This second ultrasound energy can be different than the ultrasound energy delivered by the treatment device 26. In certain embodiments, the first and second ultrasound energy can be delivered at the same time, intermittently with each other and/or with the first ultrasound energy delivered before or after the second ultrasound energy. A characterization device 103, which can be part of the control system 109 or a separate component is capable of receiving information from the transducer 86. The characterization device 103 can be electrically coupled to the transducer 86 and/or can be configured to wirelessly communicate with the transducer 86. The characterization device 103 can be configured to analyze the imaging site. In some embodiments, the characterization device 103 is a computer with the necessary electrical connections and software and/or display and input devices..

With continued reference to Figure 8, in certain embodiments, the imaging device 25 can include imaging ultrasonic element 79 electrically coupled to a control system 100 and/to characterization device (shown in Figure 1), and/or configured to emit a second ultrasonic energy that is different than the ultrasound energy delivered by the treatment device 26. In other embodiments, the imaging ultrasonic element 79 can be omitted and ultrasound from the treatment device 26 can be used for imaging.

In one embodiment, the treatment components 26 transmit a first ultrasonic energy to enhance delivery of a therapeutic compound. The imaging device 25 can, in turn, comprise a signal emitter (e.g., 79) and a signal sensor (e.g., 86). The signals can be ultrasonic energy, any imaging technology disclosed herein, or any other suitable imaging technology. The imaging device 25 can be configured to analyze a treatment site by emitting a first signal and/or sensing a second signal. The second signal can be related to the first signal. A conductor (e.g., a wire, a cable or wireless connection) can operatively couple the transducer 86 to characterization device 103, which can be part the control system 100 (shown in Figure 1).

In the embodiment described above, the imaging device is an ultrasound imaging device. However, as described above, modified embodiments can utilize, devices that use optical, IR, temperature and Doppler measurement techniques. Examples of imaging catheters and devices that used as the imaging device described herein can be found in, for example, U.S. Patent No. 8,298,147 and U.S. Patent Application 2012/0330144 of which the entirety of both are hereby incorporated by reference herein.

Figure 8A illustrates an embodiment in which the imaging device 25 includes an ultrasound element 84' that functions as the signal emitter and signal sensor. That is, in some embodiments the ultrasound element 84' can be configured to emit the second ultrasonic energy, which can be used for imaging and also for receiving the reflected second ultrasound energy which can be used for imaging. The ultrasound element 84' can be connected to the characterization device 103 and/or control unit 100 for driving the ultrasound element 84' to emit the second ultrasound energy for imaging and for receiving information from the ultrasound element 84', which can be used by the characterization device 103 for analyzing the imaging site. In certain embodiments, the first and second ultrasound energy can be delivered at the same time, intermittently with each other and/or with the first ultrasound energy delivered before or after the second ultrasound energy.

In the illustrated embodiment of Figure 1, the imaging device 25 is spaced from the treatment components 26. In such embodiments, the device may have one or more therapy areas 96 and one or more imaging areas 94. In some embodiments, the therapy areas and imaging areas can overlap such that at least a portion of the imaging area 94 can be located in the therapy area 96. In one embodiment, the ultrasonic elements of the treatment device are within 10 centimeters of the ultrasound elements of the imaging device.

In one embodiment, the imaging device 25 can aid an operator in determining of the therapeutic device 26 is properly positioned within a clot or other vascular occlusion and/or otherwise properly positioned at treatment site. The imaging device 25 can also allow the operator to identify the clot and/or determine if the device is being advanced into the clot. In this manner, the operator can determine if the tip of the catheter is being advanced into the vessel wall instead of the clot. In this manner, the operator can avoid dissecting or otherwise damaging the blood vessel. The imaging device 25can also be used to determine if the obstruction is being removed or decreasing in size during treatment The imaging device 25 can also be used to determine if treatment should be stopped and/or modified.

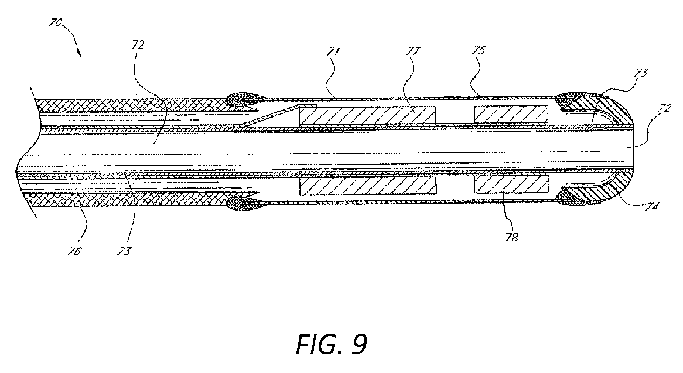

Figure 9 illustrates a cross-sectional view of an ultrasound catheter embodiment with an energy delivery (e.g., therapeutic) portion and an imaging portion, according to some embodiments. The ultrasound catheter 70 can be used to measure and/or image the targeted treatment site. In one embodiment, the catheter is an ultrasound imaging device. In other embodiments, the device can use optical sensing, Doppler, electromagnetic, and/or other techniques for gathering information about the treatment site.

The illustrated embodiment includes an optional cavitation promoting surface 71. In this embodiment, the catheter includes an inner core 73 that defines a utility lumen 72 configured to pass materials such as a guide wire, a therapeutic compound, and/or a cooling fluid. The catheter assembly 70 can further include a distal tip element 74 and a hollow cylindrical ultrasound radiating member 77 that is mounted on the inner core 73. The catheter assembly 70 can also include an outer surface 75. These components can be omitted in alternative embodiments.

In some embodiments, the ultrasound radiating member 77 illustrated in Figure 7 can be a tubular piezoceramic transducer that is able to radiate ultrasonic energy in a length mode, a thickness mode, and a circumferential mode. In some embodiments, the ultrasound radiating member 77 can be capable of generating peak acoustic pressures that are between about 0.7 MPa and about 10 MPa and/or between about 1.2 MPa and about 6 MPa.

In some embodiments, the ultrasound radiating member 77 is a first ultrasonic radiating member configured and/or used to provide ultrasonic therapy (e.g., to enhance the delivery and/or effect of a therapeutic compound). A second ultrasonic radiating member 78 can be configured and/or used to image a treatment site (e.g., to characterize the treatment site). In some embodiments, the first ultrasonic radiating member (e.g., 77) and the second ultrasonic radiating member 78 are used simultaneously. For example, the catheter assembly 70 can concurrently and/or simultaneously image and treat a target site.

The second ultrasonic radiating member 78 can be located under and/or be at least partially covered by the cavitation promoting surface 71. In some embodiments, the cavitation promoting surface 71 is located radially outward from at least a portion of the second ultrasonic radiating member 78. The cavitation promoting surface 71 can be an acoustic window. The cavitation promoting surface 71 and other portions of the catheter assembly 70 can form an enclosure that blocks bodily fluid from contacting the second ultrasonic radiating member 78. In several embodiments, the second ultrasonic radiating member 78 can be located distally, proximally, coaxially, and/or radially outward relative to the first ultrasonic radiating member 77. As with the embodiment of Figures 1-8, a conductor (e.g., a wire, a cable or wireless connection) can operatively couple the second ultrasonic radiating member 78 to a characterization device 103, which can be part the control system 100.

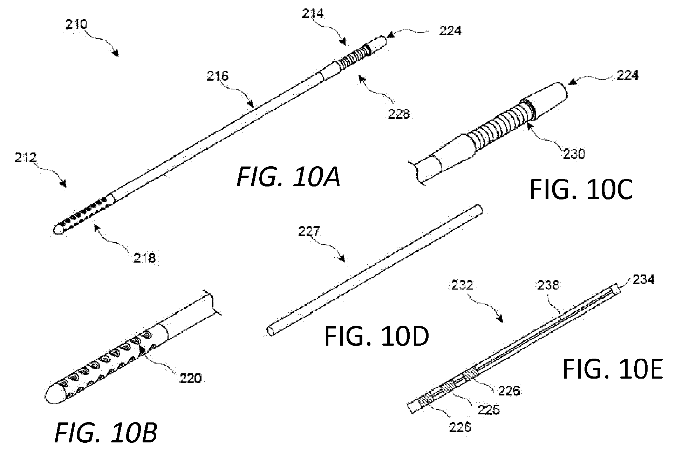

Figures 10A to 10C and Figure 10E schematically illustrate one arrangement of an ultrasonic catheter 210 with an imaging device 225 that can be used to treat a blood clot in the brain resulting from an intracerebral hemorrhage (ICH) and/or an intraventricular hemorrhage (IVH). As used herein, the term "intracranial hemorrhage" encompasses both intracerebral hemorrhage and intraventricular hemorrhage. Although some embodiments may be disclosed with reference to intracerebral hemorrhage or intraventricular hemorrhage, the embodiments can generally be used to treat both types of intracranial hemorrhages. Further details and embodiments of the catheter 210 without an imaging device 225 can be found in U.S. Patent Application 2012/0069285, the entirety of which is hereby incorporated by reference herein.

Figure 10B shows an enlarged detail view of a distal portion 212 of the catheter 210 and Figure 10C illustrates an enlarged detail view of a proximal portion 214 of the catheter 210. In the illustrated arrangement, the ultrasonic catheter 210 generally includes a multi-component, elongate flexible tubular body 216 having a proximal region 214 and a distal region 212. The tubular body 216 includes a flexible energy delivery section 218 located in the distal region 212. Within the distal region 212 are located a plurality of holes 220, through which fluid may flow into or out of a central lumen not shown that extends though the catheter 210. Although the drainage holes 220 are shown as circular, the shape of the holes may be varied.

The catheter 210 defines the hollow lumen which allows for the free flow of liquids between the drainage holes 220 and the proximal port 224. For instance, blood may flow from an area external to the ultrasonic catheter through the drainage holes 220 and into the lumen. The blood may then flow proximally in the lumen 222 towards the proximal region 214 of the ultrasonic catheter, where it may be collected via the drainage kit. In certain embodiments, any number of therapeutic compounds may be introduced into the ultrasonic catheter through the proximal end 214. The compounds, which may be dissolved or suspended within a liquid carrier, may flow through the lumen 222 and towards the distal end 212 of the ultrasonic catheter, ultimately exiting the catheter through drainage holes 220 and entering a treatment site.

In certain embodiments, negative pressure may be applied to the lumen 222 of the catheter to facilitate the flow of blood from the drainage holes 220 towards the proximal end 214. In other embodiments, no external pressure is applied, and the conditions present at the treatment site are sufficient to cause the blood to flow proximally through the lumen 222. In some embodiments, a positive pressure may be applied to the lumen 222 of the catheter 210 in order for therapeutic compounds or other liquids to pass distally through the lumen 222 towards the drainage holes 220. In other embodiments, no external pressure is applied, and the liquid is permitted to independently flow distally and exit the drainage ports 220.

The tubular body 216 and other components of the catheter 210 can be manufactured in accordance with a variety of techniques known to an ordinarily skilled artisan. Suitable materials and dimensions can be readily selected based on the natural and anatomical dimensions of the treatment site and on the desired access site. In addition, the surface of the catheter 10 can be coated with an antimicrobial material, such as silver or a silver based compound. In certain embodiments, the catheter may be biocompatible for use in the brain for up to 7 days, for up to 15 days, up to 29 days, or for up to 35 days. In one arrangement, the catheter can be coated with a hydrophilic material.

In some embodiments, the tubular body 16 can be between about 23 and 29 centimeters in length, and in other embodiments up to about 40 cm of length. In certain arrangements, the lumen has a minimum inner diameter of about 2 millimeters and the catheter body has a maximum outer diameter of about 6 mm.

In one particular embodiment, the tubular body 216 has material properties similar to that of standard external ventricular drainage (EVD) catheters. For example, the tubular body can be formed of radiopaque polyurethane or silicone, which can be provided with antimicrobial features. In such embodiments, the catheter 210 by itself may not have sufficient flexibility, hoop strength, kink resistance, rigidity and structural support to push the energy delivery section 18 through an opening in the skull and then, in turn, the patient's brain tissue to a treatment site (e.g., one of the ventricles). Accordingly, the catheter 210 can be used in combination with a stylet 227 (Figure 10D), which can be positioned within the tubular body 210. In one embodiment, the device is configured to be compatible with Neuronaviagation systems by easily accommodating the Neuronavigation system stylet. The stylet 227 can provide additional kink resistance, rigidity and structural support to the catheter 210 such that it can be advanced through the patients' brain tissue to the target site. In certain embodiments, the stylet 26 can be configured to be used in combination with a standard image guided EVD placement system. As described below, after placement, the stylet 227 can then be removed to allow drainage through the tubular body 216. In a modified arrangement, the tubular body 216 can be reinforced by braiding, mesh or other constructions to provide increased kink resistance and ability to be pushed with or without a stylet.

In one embodiment, the tubular body energy delivery section 218 can comprise a material that is thinner than the material comprising the tubular body proximal region 14. In another exemplary embodiment, the tubular body energy delivery section 18 comprises a material that has a greater acoustic transparency than the material comprising the tubular body proximal region 214. In certain embodiments, the energy delivery section 218 comprises the same material or a material of the same thickness as the proximal region 214.

Figure 10C shows an enlarged detail view of the proximal portion 214 of the ultrasonic catheter 210. The proximal portion 214 includes a connector 228. In the embodiment shown, the connector 228 comprises a series of annular rings 230 aligned in parallel. The connector 228 permits the catheter 210 to be joined to a drainage kit. For example, in one arrangement, the connector 228 is configured to connect to a standard EVD drainage kit that can include an attachment fitting that slides over the connector 228 or can include a buckle or joint that is fastened around connector 228. Specific length and configuration of the connector 228 can vary according to the needs of the particular application, and to facilitate connection with various drainage kits. Additionally, the number of annular rings 30 may vary in certain embodiments.

In the illustrated arrangement of Figures 10A-D and 10F, the catheter 200 can be used in combination with an inner core 232 (Figure 10E) which can be inserted into the lumen 222 after the stylet 227 has been removed to deliver ultrasound energy to the target site and/or to providing imaging information. The core 232 can include proximal hub 324 fitted on one end of the inner core2 32 proximal region. One or more ultrasound radiating members 226 are positioned within a distal region of the core and are coupled by wires 238 to the proximal hub 234. The core 232 can also include imaging device 225 configured in a manner similar to the imaging device described above (see e.g.,) Figure 8). In some embodiments, the inner core 232 can be inserted into the lumen 222 and/or along a side of the catheter 210. In yet another arrangement, the core 232 can be inserted into the lumen with the distal end including the ultrasound radiating members extending outside one of the holes positioned on the distal region of the catheter 210.

In one embodiment, the imaging device 225 can be used to help position the ultrasound device in a hematoma. For example, the catheter 200 can be used to treat an intracranial hemorrhage and/or a subarachnoid hemorrhage. The imaging device 225 can be used to position the device within the hematoma and to determine if the hematoma is being removed or decreasing in size during treatment. The imaging device 225 can also be used to determine if treatment should be stopped and/or modified. In one embodiment, the imaging device 225 can be incorporated into the stylet 227 and/or inserted into the catheter 200 during positioning. In yet another arrangement, the imaging device can be incorporated into the body of the catheter 200. In some embodiments, the ultrasound elements can be incorporated into the body of the catheter - see e.g. U.S. Patent Publication 2012/0059285.

Although systems and devices have been disclosed in the context of certain embodiments and examples, it will be understood by those skilled in the art that the systems and devices extend beyond the specifically disclosed embodiments to other alternative embodiments and/or uses of the embodiments and certain modifications and equivalents thereof. For example, in certain embodiments, various components are integrated and/or replaced by a single component. It should be understood that various features and aspects of the disclosed embodiments can be combined with or substituted for one another in order to form varying system modes. Accordingly, it is intended that the scope of the systems and devices herein-disclosed should not be limited by the particular disclosed embodiments described above, but should be determined only by a fair reading of current and/or future claims.

None of the steps or blocks described herein is essential or indispensable. Any of the steps and blocks can be adjusted or modified. Other or additional steps can be used. Any portion of any of the steps, processes, structures, and/or devices disclosed or illustrated in one embodiment, flowchart, or example in this specification can be combined or used with or instead of any other portion of any of the steps, processes, structures, and/or devices disclosed or illustrated in a different embodiment, flowchart, or example. The embodiments and examples provided herein are not intended to be discrete and separate from each other.

Some of the devices, systems, embodiments, and processes use computers. Each of the routines, processes, methods, and algorithms described in the preceding sections may be embodied in, and fully or partially automated by, code modules executed by one or more computers, computer processors, or machines configured to execute computer instructions. The code modules may be stored on any type of non-transitory computer-readable storage medium or tangible computer storage device, such as hard drives, solid state memory, flash memory, optical disc, and/or the like. The processes and algorithms may be implemented partially or wholly in application-specific circuitry. The results of the disclosed processes and process steps may be stored, persistently or otherwise, in any type of non-transitory computer storage such as, e.g., volatile or non-volatile storage.

Language of degree, such as the terms "approximately," "about," "generally," and "substantially," as used herein represent a value, amount, or characteristic close to the stated value, amount, or characteristic that still performs a desired function or achieves a desired result. For example, the terms "approximately," "about," "generally," and "substantially" may refer to a value, amount, or characteristic that is within less than 10% of, within less than 5% of, within less than 1% of, within less than 0.1% of, and within less than 0.01% of the stated value, amount, or characteristic. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require at least one of X, at least one of Y, and at least one of Z to each be present.

The term "and/or" means that "and" applies to some embodiments and "or" applies to some embodiments. Thus, A, B, and/or C can be replaced with A, B, and C written in one sentence and A, B, or C written in another sentence. A, B, and/or C means that some embodiments can include A and B, some embodiments can include A and C, some embodiments can include B and C, some embodiments can only include A, some embodiments can include only B, some embodiments can include only C, and some embodiments include A, B, and C. The term "and/or" is used to avoid unnecessary redundancy.

The various features and processes described above may be used independently of one another, or may be combined in various ways. All possible combinations and subcombinations are intended to fall within the scope of this disclosure. In addition, certain method, event, state, or process blocks may be omitted in some implementations. The methods and processes described herein are also not limited to any particular sequence, and the blocks or states relating thereto can be performed in other sequences that are appropriate. For example, described tasks or events may be performed in an order other than the order specifically disclosed. Multiple steps may be combined in a single block or state. The example tasks or events may be performed in serial, in parallel, or in some other manner. Tasks or events may be added to or removed from the disclosed example embodiments. The example systems and components described herein may be configured differently than described. For example, elements may be added to, removed from, or rearranged compared to the disclosed example embodiments.

## Claims

1. An ultrasound catheter system configured to provide therapeutic ultrasound and imaging ultrasound, the ultrasound catheter system comprising:
a catheter having a treatment portion;
a first ultrasonic element disposed in the treatment portion of the catheter and configured to emit a first ultrasonic energy to enhance delivery of a therapeutic compound; and
a second ultrasonic element disposed in the treatment portion of the catheter and configured to convert a second ultrasonic energy that is reflected from an imaging site into an imaging signal.

2. The ultrasound catheter system of Claim 1, further comprising a control system electrically coupled to the first ultrasonic element, wherein the control system is configured to drive the first ultrasonic element to generate the first ultrasonic energy to enhance the delivery of the therapeutic compound.

3. The ultrasound catheter system of Claim 1 or 2, wherein the second ultrasonic element comprises a transducer, and the ultrasound catheter system further comprises a characterization device capable of receiving information from the transducer, wherein the characterization device is configured to analyze the imaging site.

4. The ultrasound catheter system of any one of Claims 1-3, further comprising a therapy area and an imaging area, wherein the first ultrasonic element is configured to enhance the delivery of the therapeutic compound in the therapy area and the second ultrasonic element is configured to enable imaging the imaging area, and wherein at least a portion of the imaging area is located in the therapy area.

5. The ultrasound catheter system of any one of Claims 1-4, wherein the first ultrasonic element comprises a first piezoelectric element and the second ultrasonic element comprises a second piezoelectric element.

6. The ultrasound catheter system of any one of Claims 1-5, further comprising a third ultrasonic element disposed in the distal portion of the catheter, electrically coupled to a control system, and configured to emit the second ultrasonic energy.

7. The ultrasound catheter system of any one of Claim 1, wherein the second ultrasound element is configured to emit the second ultrasonic energy.

8. The ultrasound catheter system of Claim 7, wherein the second ultrasound element is coupled to a control system that is configured to drive the second ultrasound element to emit the second ultrasonic energy.

9. The ultrasound catheter system of Claim 7 or 8, wherein the ultrasound catheter system further comprises a characterization device capable of receiving information from the second ultrasound element, wherein the characterization device is configured to analyze the imaging site.

10. An ultrasound catheter system configured to provide therapeutic ultrasound, the ultrasound catheter system comprising:
a catheter having a treatment portion;
a therapeutic assembly comprising a first ultrasonic element disposed in the treatment portion of the catheter and configured to emit a first ultrasonic energy to enhance delivery of a therapeutic compound;
an imaging assembly comprising a signal emitter and a signal sensor, wherein the signal emitter and the signal sensor are disposed in the treatment portion of the catheter, and wherein the imaging assembly is configured to analyze a treatment site by emitting a first signal and sensing a second signal.

11. A method of operating an ultrasonic catheter comprising:
advancing a catheter comprising a treatment portion having a first ultrasonic element and an imaging device to a treatment site;
delivering of a therapeutic compound and ultrasound energy to the treatment site; and
imaging the treatment site.

12. The method of Claim 11, wherein imaging the treatment site comprises emitting a ultrasonic energy towards the treatment site, reflecting at least a portion of the ultrasonic energy from the treatment site, and converting the ultrasonic energy into an electrical signal.

13. The method of Claim 10 or 11, wherein the imaging the treatment site occurs before delivering of a therapeutic compound and ultrasound energy to the treatment site.
